# EUROPEAN PATENT APPLICATION

(11) **EP 4 494 460 A1**
(43) Date of publication of application: **22.01.2025**
(21) Application number: 23769543.2
(22) Date of filing: 24.02.2023
(51) Int. Cl.: A01H 1/02, A01H 1/08, A01H 1/04, A01H 4/00

(54) **SELF-INCOMPATIBLE DIHAPLOID POTATO LINE AND BREEDING METHOD THEREFOR, AND HYBRID POTATO AND SEED PRODUCTION METHOD THEREFOR**

(30) Priority: 16.03.2022 CN 202210258745
(71) Applicant: Agricultural Genomics Institute at Shenzhen, Chinese Academy of Agricultural Sciences, Shenzhen, Guangdong 518124 (CN)
(72) Inventor: ZHANG, Chunzhi, Shenzhen, Guangdong 518124 (CN); HUANG, Sanwen, Shenzhen, Guangdong 518124 (CN); WANG, Pei, Shenzhen, Guangdong 518124 (CN); ZHU, Yanhui, Shenzhen, Guangdong 518124 (CN); JING, Xinyu, Shenzhen, Guangdong 518124 (CN)
(74) Representative: Murgitroyd & Company
(86) International application number: PCT/CN2023/078207
(87) International publication number: WO 2023/174035

(57) **Abstract**

The present application relates to a self-incompatible dihaploid potato and a production method therefor, and a hybrid potato and a seed production method therefor. The production method includes the following steps: haploid-inducing a diploid potato carrying one or more heterozygous self-compatibility genes by a haploid induction line, and screening haploid mutants; selecting a haploid mutant which does not contain any self-compatible gene; and performing chromosome doubling on the haploid mutant which does not contain any self-compatibility gene to obtain a self-incompatible dihaploid potato line. The self-incompatible dihaploid potato is used as a female parent for seed production, such that emasculation can be avoided, and hybrids do not bear fruits, thereby improving the yield of potato tubers.

## Description

This application claims the priority of the Chinese patent application with application number 202210258745.2 which is submitted to the Chinese Patent Office on March 16, 2022, and titled "Self-incompatible Dihaploid Potato Line and Production Method Therefor, and Hybrid Potato and Seed Production Method Therefor ", which is incorporated herein by reference in its entirety.

### FIELD OF THE INVENTION

The present invention relates to the field of breeding technology, and in particular to a self-incompatible dihaploid potato line and production method therefor, and hybrid potato and seed production method therefor.

### BACKGROUND OF THE INVENTION

Potatoes are the fourth largest staple food in the world. However, compared with crops such as rice and corn, the genetic gain of potatoes is limited, mainly due to its tetraploid characteristics. Cultivated potatoes are mainly autotetraploid, and their genomes are highly heterozygous, thereby making genetic analysis difficult, and resulting in long breeding cycles and slow variety update. In addition, the existing potatoes mainly use potato tubers for vegetative propagation, which has problems such as low reproduction coefficient, high storage and transportation costs, and vulnerability to diseases and insect pests. To solve the structural obstacles faced by the potato industry, many scientists have called for hybrid potato breeding, i.e., breeding with diploids instead of tetraploids to speed up the breeding process, and reproducing with hybrid seeds instead of tubers to reduce production costs and the spread of insect pests. Therefore, hybrid breeding will revolutionize potato breeding and reproduction modes (Jansky et al., 2016; Lindhout et al., 2011; Stokstad, 2019; Zhang et al., 2021).

The main process of hybrid potato breeding is: firstly, selecting suitable diploid starting materials to break self-incompatibility, then obtaining an inbred line with highly homozygous genome through multi-generation self-crossing, and finally hybridizing the different inbred lines to generate F₁ hybrid combinations, and select hybrids with excellent traits. Since the inbred lines as hybrid parents are self-compatible, when hybridizing different inbred lines, the female parent needs to be emasculated to prevent the self-crossed seeds from the female parent from being mixed into the hybrid seeds, resulting in insufficient purity of the hybrid seeds and affecting the commercial nature. At present, due to the self-compatibility characteristics of potato inbred lines, stigmas are easily contaminated with stamen pollen, and artificial emasculation is still used in potato hybrid seed production. No publicly reported method may avoid emasculation during hybrid seed production and prevent the production of fruit in hybrids. The emasculation process of potato emasculation is very time-consuming and labor-intensive, resulting in high hybrid production costs. Moreover, there are certain deviations in manual emasculation, which may lead to untimely or incomplete emasculation, resulting in a certain false hybrid rate. In addition, hybrids produced by crossing different inbred lines are also self-compatible. In the actual production process, the above-ground parts will naturally produce large amounts of fruit, and the above-ground fruits and underground tubers compete for nutrients, which affects the yield of underground tubers to a certain extent (Zhang et al., 2021). Therefore, it is very necessary and urgent to find a method that does not require emasculation during the seed production process, and the hybrids do not bear fruit.

### BRIEF DESCRIPTION OF THE INVENTION

Given this, the present disclosure provides a self-incompatible dihaploid potato line and a production method therefor, and a hybrid potato and a seed production method therefor. Using this self-incompatible dihaploid potato as a female parent for seed production, not only emasculation may be avoided, but also fruitless hybrids may be obtained, thereby increasing the yield of potato.

To achieve the above-mentioned object of the disclosure, the present disclosure provides the following technical solutions:
The disclosure provides a method for the production of a self-incompatible dihaploid potato line, the method includes the following steps:
(1) haploid-inducing a diploid potato carrying one or more heterozygous self-compatibility genes by a haploid induction line, and screening haploid mutants;
(2) selecting a haploid mutant which does not contain any self-compatible gene; and
(3) performing chromosome doubling on the haploid mutant which does not contain any self-compatibility gene to obtain a self-incompatible dihaploid potato line.

Before this disclosure, since too many harmful mutations had accumulated in potatoes and the efficiency was too low, breeders mainly relied on the scheme of obtaining viable homozygous lines through selfing, and self-incompatibility regarded as a disadvantage trait for breeding was broken so that homozygous lines may be obtained by selfing. The present disclosure breaks out of the inherent thinking and flexibly utilizes the self-compatibility and incompatibility traits of potatoes at different stages of production, and the final combination creates a simple and efficient method for potato seed production that may increase potato tuber yield.

In a particular embodiment provided by the present disclosure, self-compatibility genes include but are not limited to, S_{S11} gene and/or Sli gene.

In a particular embodiment provided by the present disclosure, the diploid potato with heterozygous self-compatibility genes is a diploid potato containing two self-compatibility genes in a heterozygous state. However, the present disclosure is not limited thereto. Diploid potatoes containing one or more heterozygous self-compatibility genes may achieve the technical effects of the present disclosure.

In a particular embodiment provided by the present disclosure, a haploid induction line includes but is not limited to, a haploid induction line obtained by utilizing haploid induction (HI) genes such as PLA1/MTL/NLD and DMP.

Particularly, the haploid induction line includes but is not limited to haploid induction line PL4 and/or haploid induction line obtained by using the potato StDMP gene. The potato StDMP gene is an abbreviation of Soltu.DM.05G005100.1 gene (detailed information may be found at Spud DataBase http://spuddb.uga.edu/). The haploid induction line obtained by using the potato StDMP gene is described in CN202210109081.3.

Preferably, methods for screening haploid mutant include one or more of a seed appearance characteristic marker, a molecular marker, a fluorescent marker, and flow cytometry.

In a particular embodiment provided by the present disclosure, a seed appearance characteristic marker is the presence or absence of embryonic spots. If seeds have embryonic spots, the seeds are diploid hybrids and need to be eliminated; if seeds have no embryonic spots, they may be haploid and need to be retained for further testing. However, seed appearance characteristic markers are not limited to these. Appearance characteristic markers recognized by those skilled in the art are all within the protection scope of the present disclosure.

Concerning methods for screening haploid mutant by using a molecular marker, a fluorescent marker, or flow cytometry, can refer to the relevant contents disclosed in Zhong et al., 2020 (Zhong, Y., et al." A DMP-triggered in vivo maternal haploid induction system in the dicotyledonous Arabidopsis." Nature Plants 6.5(2020):466-472) and/or CN202210109081.3.

In the present disclosure, a diploid potato carrying one or more heterozygous self-compatibility genes is used as the female parent. The female parent is self-compatible and may produce a certain probability of selfing seeds due to untimely or incomplete emasculation. These seeds also do not contain embryonic spots and may be eliminated using molecular markers. In a particular embodiment provided by the present disclosure, the primer sequences of molecular markers include:
1) chr01_623388:
   Forward primer: 5'-TACAAATATTCAGCGAAGGG-3';
   Reverse primer: 5'-GCTCAATTGTCTTCTTCAATCC-3'.
2) chr02_112634:
   Forward primer: 5'-TCCTCCATCTCAGATTCACT-3';
   Reverse primer: 5'-GGGTACGATTGTGAACATCT-3'.
3) chr03_265493:
   Forward primer: 5'-CTATAATTTGGGCACGTGAT-3';
   Reverse primer: 5'-CTAGTTGCATTCAGCCTTCT-3'.
4) chr06_537151:
   Forward primer: 5'-CAGAGACAGGAACAGAAAATG-3';
   Reverse primer: 5'-TGAAGGGACTAATTTGGCTA-3'.
5) chr07_377300:
   Forward primer: 5'-GGGATCATTTCGATCTTTCT-3';
   Reverse primer: 5'-GTAAATTGGAAATCCCTCCT-3'.
6) chr08_844961:
   Forward primer: 5'-TGACCCTTTGGATGAAATAC-3';
   Reverse primer: 5'-CACCTTATCAAACGAAGCTC-3'.

However, molecular markers used to eliminate seeds obtained from maternal self-pollination are not limited to above markers. As long as molecular markers may identify seeds obtained from maternal self-pollination, they are within the protection scope of the present disclosure.

In step (2) of the present disclosure, a method for selecting a haploid mutant that does not contain any self-compatible gene includes: detecting the presence of a self-compatible gene by using primers specific for self-compatible genes.

In a particular embodiment provided by the present disclosure, the S_{S11} gene-specific primers are as follows:
Forward primer: 5'-GAAGAAAGGAAATGAAGTGAGTTGTTC-3';
Reverse primer: 5'-GAATAATAACTTATTTCTTGATGGGATTG-3'

In a particular embodiment provided by the present invention, the Sli gene-specific primers are as follows:
Forward primer: 5'-CGTCGGATTCAGCAGCAGAGTT-3';
Reverse primer: 5'-AAGCGAATTACAAGCCTGTTTAGATTGAC-3'

In a particular embodiment provided by the present disclosure, the haploid mutants are selected from one or more of seeds, plants, and tissue cells.

In a particular embodiment provided by the present disclosure, tissue cells are selected from one or more types of buds, stem segments, roots, leaves, and flowers.

In a particular embodiment provided by the present disclosure, the haploid mutants in step (1) are seeds or plants.

In a particular embodiment provided by the present disclosure, the haploid mutant in step (2) is a seed or a plant.

In a particular embodiment provided by the present disclosure, the haploid mutant in step (3) is a tissue cell, a bud, or a plant.

Preferably, methods for chromosome doubling include but are not limited to a chemical induction method, a low-temperature induction method, a callus regeneration method, etc.

Preferably, the method for chromosome doubling is a chemical induction method.

The chemical induction method includes but is not limited to treating the haploid mutant with one or more of colchicine, cytochalasin B, and/or PMTS (phenylmercury-p-toluene sulfonilide); preferably treating the haploid mutant with colchicine.

In a particular embodiment provided by the present disclosure, the treatment concentration of colchicine is 0.1-1%.

In a particular embodiment provided by the present disclosure, the treatment concentration of colchicine is 0.35-0.55%.

In a particular embodiment provided by the present disclosure, the treatment time of colchicine is at least 2 days.

The present disclosure also provides a self-incompatible dihaploid potato line, and a plant of the dihaploid potato line does not contain any self-compatibility gene and is fertile.

In a particular embodiment provided by the present disclosure, the plants of the dihaploid potato line further include one or more beneficial alleles. Preferably, the beneficial alleles include Floral Bud Abortion 1 (FBA1) allele (with fertility), and/or Yellow (Y) allele (with yellow tuber flesh).

In a particular embodiment provided by the present disclosure, the plants of the dihaploid potato line further do not contain one or more harmful alleles. Preferably, the harmful alleles include floral bud abortion 1(fba1) allele (with infertility), and/or large-effect deleterious mutation 1 (led1) allele (affecting survival).

In a particular embodiment provided by the present disclosure, the dihaploid potato line may be obtained by the above-mentioned production method.

In a particular embodiment provided by the present disclosure, the dihaploid potato lines include but are not limited to one or more of YS1-187, YS1-192, YS1-311, and YS1-366.

The present disclosure also provides a plant of the above-mentioned dihaploid potato line, plant part thereof, tuber or tuber part, or plant cell, pollen, or seed thereof.

The present disclosure also provides a seed production method for obtaining a hybrid potato, wherein a plant of the dihaploid potato line obtained by the above production method or a plant of the above-mentioned dihaploid potato line is used as a female parent for hybrid seed production.

In a particular embodiment provided by the present disclosure, wherein a male parent for hybrid seed production is selected from a plant of the dihaploid potato line obtained by the above production method, a plant of the above dihaploid potato line, or a plant of other self-incompatible potato lines.

In another particular embodiment provided by the present disclosure, a male parent for hybrid seed production is a plant of a self-compatible potato line.

The present disclosure also provides a hybrid potato seed obtained by the above seed production method, plant grown from the seed, plant part thereof, tuber or tuber part, or plant cell, pollen, or seed thereof.

The present disclosure also provides feed or food product including materials from the above hybrid potato plant, plant part thereof, tuber or tuber part, or the plant cell, pollen, or seed thereof.

Particularly, the feed includes but is not limited to liquid feed, solid feed, semi-solid feed, or feed raw materials; and the food includes but is not limited to fresh potatoes, dried potatoes, frozen potatoes, French fries, potato chips, potato flour, or potato starch.

The present disclosure also provides a method for manufacturing a commercial plant product, including obtaining the above-mentioned plants, the plant parts, tubers, or tuber parts thereof to manufacture the commercial plant product, wherein the plant products are selected from the group consisting of fresh potato material, frozen potato material, dehydrated potato material, potato liquid, French fries, potato chips, potato granules, potato flour, and potato starch.

Compared with the prior art, the technical effects of the present disclosure are as follows:
(1) the present disclosure obtains self-incompatible and fertile dihaploid potato material for the first time;
(2) using the self-incompatible dihaploid material obtained by the method according to the present disclosure as the female parent for hybridization, artificial emasculation may be avoided, and the cost of seed production for hybrid potato lines is reduced while ensuring hybrid rate; and
(3) using the self-incompatible dihaploid materials obtained by the method of the present disclosure as a parent for hybridization, not only may artificial emasculation be avoided and the hybrid rate be increased, but also the problem of fruit-bearing of the above-ground parts is also avoided, thereby improving the yield of potatoes.

### BRIEF DESCRIPTION OF THE DRAWINGS

Fig. 1 is a schematic diagram of the production of self-incompatible dihaploid potato;
Fig. 2 shows a comparison between seeds without embryo spots and seeds with embryo spots;
Fig. 3 shows self-incompatible haploid plants and diploid plants.

### BRIEF DESCRIPTION OF THE INVENTION

The present disclosure discloses a self-incompatible dihaploid potato and a production method therefor, and a hybrid potato and a seed production method therefor. Persons skilled in the art may learn from the contents of this disclosure, and appropriately improve the process parameters for implementation. It should be noted that all similar substitutions and modifications are obvious to those skilled in the art, and they are deemed to be encompassed in the present disclosure. The methods and applications of the present disclosure have been described through preferred embodiments. Relevant persons may make modifications or appropriate changes and combinations to the methods and applications described herein without departing from the contents, principle, and scope of the present disclosure to achieve and apply the technology of this disclosure.

The disclosure provides a method for avoiding emasculation during seed production and inhibiting fruit-bearing of the above-ground parts of a hybrid potato, the method mainly includes the following steps.
(1) Haploid-inducing a diploid potato carrying one or more heterozygous self-compatibility genes by a haploid induction line, and screening haploid mutants.
   Diploid potatoes carrying one or more heterozygous self-compatibility genes were selected for haploid induction. Generally, diploid potatoes are directly induced into haploids, and the haploids obtained are difficult to survive mainly due to carrying harmful mutations with large effects. As for diploid materials carrying self-compatibility genes, some harmful mutations with large effects may have been eliminated through selfing. These diploid materials that have removed harmful mutations with large effects are selected for performing haploid induction to obtain haploid mutants with a higher survival probability.
(2) Selecting a single plant which does not contain any self-compatible gene from the induced haploid mutants, and performing chromosome doubling to obtain a dihaploid potato plant which is self-incompatible.
   A single plant which does not contain any self-compatible gene is selected from the induced haploid mutants. A diploid material carrying one or more heterozygous self-compatibility genes is used for haploid induction, and there is a separation of self-compatible and self-incompatible mutants. A self-incompatible single plant screened by molecular marker is chromosome-doubled to obtain a dihaploid material having a homozygous genome but being self-incompatible, i.e., a dihaploid line.
(3) Using the above self-incompatible dihaploid material as a parent for hybridization, emasculation may be avoided, and the resulting hybrids are also self-incompatible.

Using the dihaploid plant obtained from steps (1)-(2) as a female parent to hybridize with other parents, the problem of emasculation may be avoided. This step is only limited to the fact that the female parent is self-incompatible, and emasculation is not needed.

The above steps (1)-(2) are used to create dihaploid materials with different genetic backgrounds. Crossbreeding between these materials for hybrid seed production not only avoids emasculation but also produces the resulting hybrid offspring that are also self-incompatible, thereby avoiding the problem of the fruit-bearing of the above-ground parts affecting the underground yield. In this step, both the female and male parents are self-incompatible, thereby simplifying the seed production process and increasing the yield.

### Terminology Explanation:

Allele: refers to a gene located at the same position on a pair of homologous chromosomes that controls a relative trait. An individual developing from a zygote formed by the union of gametes of the same gene is called a homozygote (such as AA and aa), while an individual developing from a zygote formed by the union of gametes of different genes is called a heterozygote (such as Aa).

Self-incompatibility: refers to the self-sterility of a plant that has complete flowers and may form normal male and female gametes, but lacks the ability to self-pollinate and set fruit.

Fertile: refers to a plant or seed that, when grown into a plant, has complete flowers and can form normal female and male gametes, and can be used as a male or female parent to produce offspring through fertile pollen or fertile ovules (i.e. male flowers and female flowers are all viable).

Chromosome ploidy: refers to the number of chromosome complements or genomes contained in a cell. The number of chromosomes contained in normal gametocytes or half of the number of chromosomes in somatic cells is called a set of haploid chromosomes, represented by the symbol n. The complete set of haploid chromosomes of an organism is called the genome or chromosome complement of that organism. Cells with one chromosome complement and individuals composed of such cells are called haploidy (n). Cells or individuals with two chromosome complements are called diploidy (2n). Cells or individuals with more than two chromosome complements are called polyploidy, including triploidy (3n), tetraploidy (4n), etc. Polyploidy formed from chromosome complements from the same source is called autopolyploidy, and polyploidy formed from different chromosome complements from different sources is called allopolyploidy.

Dihaploid (double haploid): refers to cells or individuals obtained by conducting chromosome doubling on haploid, a dihaploid usually has a high genomic homozygosity rate (≥99.99%).

Hybridization: refers to the crossing of two individuals with different genotypes, also refers to mating between different varieties, for example, cross-pollination between different plant varieties.

Selfing: refers to the crossing of two individuals with the same genotype, for example, self-pollinating of plants.

Male parent: refers to one of the parents participating in the hybridization. In animals and plants, it is a male individual or an individual that produces male germ cells.

Female parent: herein refers to a plant that receives pollen when flowers of different plants undergo cross-pollination. During plant reproduction, it refers to the female plant of the previous generation.

The experimental methods in the following examples are all conventional methods unless otherwise specified.

The biological materials, sequences, reagents or instruments used in the examples of the present invention can all be purchased from the market.

The present invention will be further described below in conjunction with the following examples.

### Example 1: Creation of Self-incompatible Dihaploid Materials

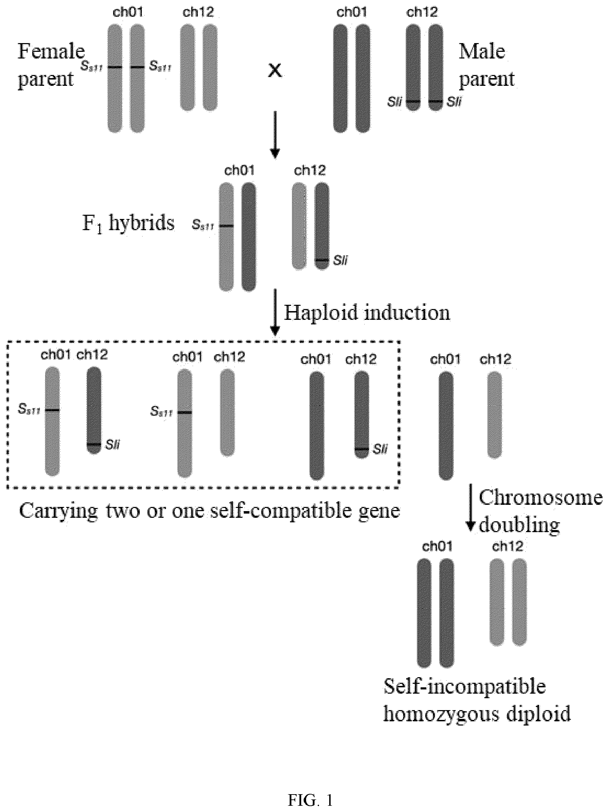

The male parent and female parent of hybrid potato "Upotato 1" (Cell, 184:3873-3883, 2021, hybrid H1 of A6-26 and E4-63) were selected for hybridization to produce F₁ hybrids. The female parent and male parent contain different self-compatibility genes respectively. The female parent contains the S_{S11} gene located on chromosome 1, and the male parent contains the Sli gene located on chromosome 12. In the F₁ hybrid, the two genes are in a heterozygous state (see Fig. 1).

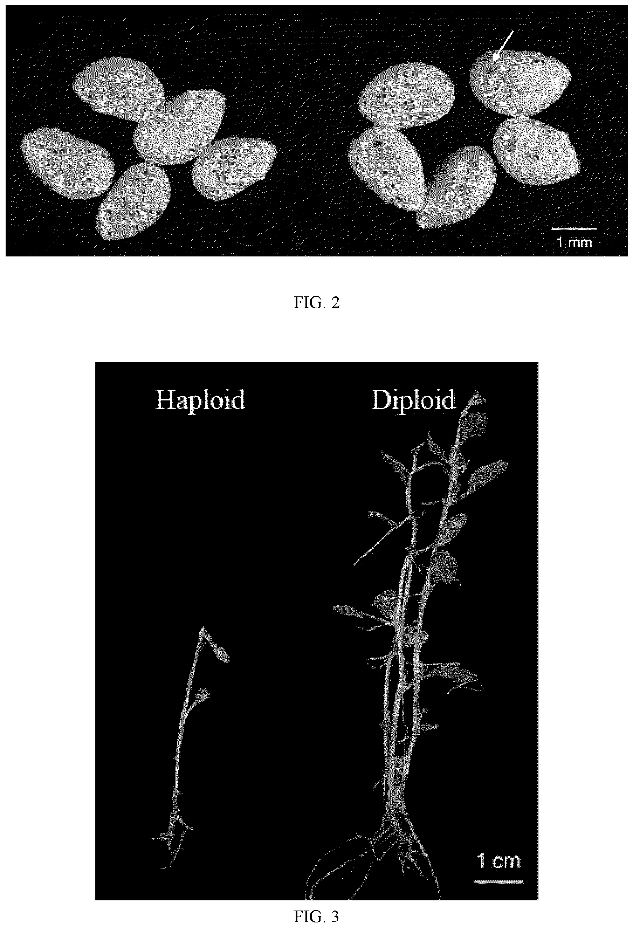

The haploid induction line PL4 introduced from the United States Department of Agriculture was used (other haploid induction lines may also be used) to haploid induce (pollinate into) the obtained hybrids, then screening haploid mutants. The screening of the haploid mutants is mainly divided into three steps.
(1) Eliminating seeds with purple embryonic spots. Since male parent PL4 is homozygous for purple embryonic spots (see Fig. 2), and the seeds of the female parent do not have embryonic spots, theoretically the seeds produced after hybridization should have purple embryonic spots. If not, it means that it may be a haploid mutant developing from a female gamete of the female parent.
(2) Using molecular markers to detect whether seeds without embryonic spots are obtained by selfing. Since the female parent is self-compatible, there may be a certain probability of selfing seeds due to untimely or incomplete emasculation. These seeds also do not have embryonic spots, and they may be eliminated through molecular markers. The primer sequences of the molecular markers include:
   1) chr01_623388:
      Forward primer: 5'-TACAAATATTCAGCGAAGGG-3';
      Reverse primer: 5'-GCTCAATTGTCTTCTTCAATCC-3'.
   2) chr02_112634:
      Forward primer: 5'-TCCTCCATCTCAGATTCACT-3';
      Reverse primer: 5'-GGGTACGATTGTGAACATCT-3'.
   3) chr03_265493:
      Forward primer: 5'-CTATAATTTGGGCACGTGAT-3';
      Reverse primer: 5'-CTAGTTGCATTCAGCCTTCT-3'.
   4) chr06_537151:
      Forward primer: 5'-CAGAGACAGGAACAGAAAATG-3';
      Reverse primer: 5'-TGAAGGGACTAATTTGGCTA-3'.
   5) chr07_377300:
      Forward primer: 5'-GGGATCATTTCGATCTTTCT-3';
      Reverse primer: 5'-GTAAATTGGAAATCCCTCCT-3'.
   6) chr08_844961:
      Forward primer: 5'-TGACCCTTTGGATGAAATAC-3';
      Reverse primer: 5'-CACCTTATCAAACGAAGCTC-3'.
(3) using flow cytometry to detect the chromosome ploidy of suspected haploid plants and ultimately determine which plants are haploid.

The above method was used to screen out 17 haploid plants from 36,801 hybrid seeds. Subsequently, two primers respectively specific to self-compatibility genes S_{S11} and Sli were used to detect the presence of the two genes, and it was found that 4 individual plants do not contain these two genes, and they are respectively numbered YS1-187, YS1-192, YS1-311, and YS1-366.

S_{S11} gene-specific primers:
Forward primer: 5'-GAAGAAAGGAAATGAAGTGAGTTGTTC-3';
Reverse primer: 5'-GAATAATAACTTATTTCTTGATGGGATTG-3'

Sli gene-specific primers:
Forward primer: 5'-CGTCGGATTCAGCAGCAGAGTT-3';
Reverse primer: 5'-AAGCGAATTACAAGCCTGTTTAGATTGAC-3'

Then the top shoots of the haploid plants were taken to conduct chromosome doubling treatment through 0.35-0.55% colchicine for at least 2 days, thereby obtaining dihaploid plants (see Fig. 3). These dihaploid plants were transplanted to the greenhouse and self-pollinated during flowering. It was found that they did not bear fruits finally, proving that they were indeed self-incompatible. Through the above process, self-incompatible dihaploid plants were finally obtained, and they can be used for hybrid potato breeding.

### Example 2: Using Self-incompatible Parents for Seed Production to Increase the Rate of Hybrids

Hybridization was conducted by using the self-incompatible dihaploid material obtained in Example 1 as the female parent, and the self-compatible homozygous diploid material D180 [D180 was obtained from the hybridization of CIP 703280 and E172 (Cell, 184:3873-3883, 2021)] as the male parent. The female parent will not be emasculated during pollination. In the control group, the female parent A6-26 (A6-26 was obtained from the selfing of CIP 705468) and the male parent E4-63 [E4-63 was obtained from the hybridization of CIP 703767 and E172 (Cell, 184:3873-3883, 2021)] were both self-compatible homozygous diploid materials, and the female parent is artificially emasculated during the hybridization process.

Enough hybrids were obtained by using the above two methods, but the first method is more time-saving and labor-saving. For each method, 500 seeds were selected for purity testing of the hybrids through molecular markers. The results prove that the rate of hybrids in the first method is 100%, and that in the second method is 97.5%. Therefore, using self-incompatible dihaploid materials as female parents for hybrid seed production can not only avoid emasculation during the hybridization process, save manpower, but also increase the rate of the hybrids.

The above contents are only preferred embodiments of the present invention. It should be noted that, those skilled in the art can make several improvements and modifications without departing from the principles of the present invention. These improvements and modifications should also be regarded as falling in the protection scope of the present invention.

## Claims

1. A method for the production of a self-incompatible dihaploid potato line, **characterized in that** it comprises the following steps:
(1) haploid-inducing a diploid potato carrying one or more heterozygous self-compatibility genes by a haploid induction line, and screening haploid mutants;
(2) selecting a haploid mutant which does not contain any self-compatible gene; and
(3) performing chromosome doubling on the haploid mutant which does not comprise any self-compatibility gene to obtain a self-incompatible dihaploid potato line.

2. The production method according to claim 1, **characterized in that** the one or more self-compatibility genes comprises S_{S11} gene and/or Sli gene.

3. The production method according to claim 1 or 2, **characterized in that** the method for screening haploid mutant comprises one or more of: a seed appearance characteristic marker, a molecular marker, a fluorescent marker, and flow cytometry.

4. The production method according to claim 3, **characterized in that** the seed appearance characteristic marker comprises the presence or absence of embryonic spots.

5. The production method according to any one of claims 1-4, **characterized in that** the haploid mutants are one or more of: seeds, plants, and tissue cells.

6. The production method according to any one of claims 1-5, **characterized in that** the method for chromosome doubling comprises one or more of: a chemical induction method, a low temperature induction method, and a callus regeneration method;
preferably the method for chromosome doubling is a chemical induction method;
the chemical induction method comprises treating the haploid mutant with one or more of: colchicine, cytochalasin B and/or PMTS (phenylmercury-p-toluene sulfonilide);
preferably treating the haploid mutant with colchicine.

7. A self-incompatible dihaploid potato line, **characterized in that** a plant of the dihaploid potato line does not comprise any self-compatibility gene, and the plant is fertile.

8. The dihaploid potato line according to claim 7, **characterized in that** the plant of the dihaploid potato line further comprises one or more beneficial alleles, preferably the beneficial alleles comprise: Floral Bud Abortion 1 (FBA1) allele, and/or yellow (Y) allele.

9. The dihaploid potato line according to claim 7 or 8, **characterized in that** the plant of the dihaploid potato line further does not comprise one or more harmful alleles, preferably the harmful alleles comprise: floral bud abortion 1 (fba1) allele, and/or large-effect deleterious mutation 1 (led1) allele.

10. The dihaploid potato line according to any one of claims 7-9, **characterized in that** it is obtained by the production method according to any one of claims 1-6.

11. The dihaploid potato line according to any one of claims 7-10, **characterized in that** the line comprises one or more of: YS1-187, YS1-192, YS1-311, and YS1-366.

12. A plant of the dihaploid potato line according to any one of claims 7-11, its plant parts, tubers or tuber parts, or its plant cells, pollens or seeds.

13. A seed production method for obtaining a hybrid potato, **characterized in that** a plant of the dihaploid potato line obtained by the production method according to any one of claims 1-6 or a plant of the dihaploid potato line according to any one of claims 7-11 is used as a female parent for hybrid seed production.

14. The seed production method according to claim 13, **characterized in that** a male parent for hybrid seed production is selected from: a plant of the dihaploid potato line obtained by the production method according to any one of claims 1-6, a plant of the dihaploid potato line according to any one of claims 7-11, or a plant of other self-incompatible potato line;
alternatively, the male parent of the hybrid seed production is a plant of a self-compatible potato line.

15. Hybrid potato seeds obtained by the seed production method according to claim 13 or 14, the plants grown from the seeds, the plant parts, tubers or tuber parts thereof, or plant cells, pollen or seeds thereof.

16. A feed or food product comprising materials from the hybrid potato plant, the plant parts, tubers or tuber parts thereof, or the plant cells, pollens or seeds thereof according to claim 15.

17. The feed or food product according to claim 16, **characterized in that** the feed comprises: liquid feed, solid feed, semi-solid feed or feed raw materials; and the food comprises: fresh potatoes, dried potatoes, frozen potatoes, French fries, potato chips, potato flour or potato starch.

18. A method for manufacturing a commercial plant product, **characterized in that** comprising:
obtaining a plant according to claim 15, the plant parts, tubers or a tuber parts thereof to manufacture the commercial plant product, wherein the plant products are selected from the group consisting of: Fresh potato material, frozen potato material, dehydrated potato material, potato liquid, French fries, potato chips, potato granules, potato flour, and potato starch.
